(19) Europäisches Patentamt
European Patent Office
Office européen des brevets

(11) Publication number: 0 440 219 A1

## (12) EUROPEAN PATENT APPLICATION

(21) Application number: 91101278.9

(22) Date of filing: 31.01.91

(51) Int. Cl.5: **C12N 15/45**, C12N 15/47,
C12N 5/10, A61K 39/165,
A61K 39/205, C12N 7/00

(30) Priority: 02.02.90 EP 90102120

(43) Date of publication of application:
07.08.91 Bulletin 91/32

(84) Designated Contracting States:
AT BE CH DE DK ES FR GB GR IT LI LU NL SE

(71) Applicant: SCHWEIZ. SERUM- & IMPFINSTITUT
BERN
Postfach 2707
CH-3001 Bern(CH)

(72) Inventor: Billeter, Martin A., Prof. Dr.
Balgriststrasse 70
CH-8008 Zürich(CH)
Inventor: Cattaneo, Roberto

Schauenbergstrasse 29
CH-8046 Zürich(CH)
Inventor: Schmid, Anita
Allenmoosstrasse 123
CH-8057 Zürich(CH)
Inventor: Ballart, Isidro
Kreuzwiesen 12
CH-8050 Zürich(CH)
Inventor: Eschle, Daniel
Leonhardhalde 15
CH-8001 Zürich(CH)

(74) Representative: Vossius & Partner
Siebertstrasse 4 P.O. Box 86 07 67
W-8000 München 86(DE)

(54) cDNA corresponding to the genome of negative-strand RNA viruses, and process for the production of infectious negative-strand RNA viruses.

(57) The present invention relates, in general, to a methodology suitable for generating infectious negative-stranded viruses, suitable for use as vaccines, from cloned complementary deoxyribonucleic acid (cDNA). The invention relates specifically to cDNA molecules and to helper cells suitable as tools in this methodology and to the characterization of the resulting viruses. Measles virus (MV) is used as a model for negative-strand RNA viruses. The described invention provides the technology for construction of recomibnant MV vaccine strains encoding epitopes or entire proteins from heterologous viruses, bacteria, or parasites. With slight modification of the technology described, any negative-stranded RNA virus can by used as vector in place of MV.

Figure 3

# CDNA CORRESPONDING TO THE GENOME OF NEGATIVE-STRAND RNA VIRUSES, AND PROCESS FOR THE PRODUCTION OF INFECTIOUS NEGATIVE-STRAND RNA VIRUSES

The present invention relates, in general, to a methodology suitable for generating infectious negative-strand RNA viruses, suitable for use as vaccines, from cloned complementary deoxyribonucleic acid (cDNA). The invention also relates to cDNA molecules suitable as tools in this methodology. Measles virus (MV) is used as a model for negative-strand RNA viruses. The described invention provides the technology for construction of recombinant MV vaccine strains encoding epitopes or entire proteins from heterologous viruses, bacteria or parasites. The method described hereafter can be also applied in principle to any other negative-strand RNA virus instead of MV.

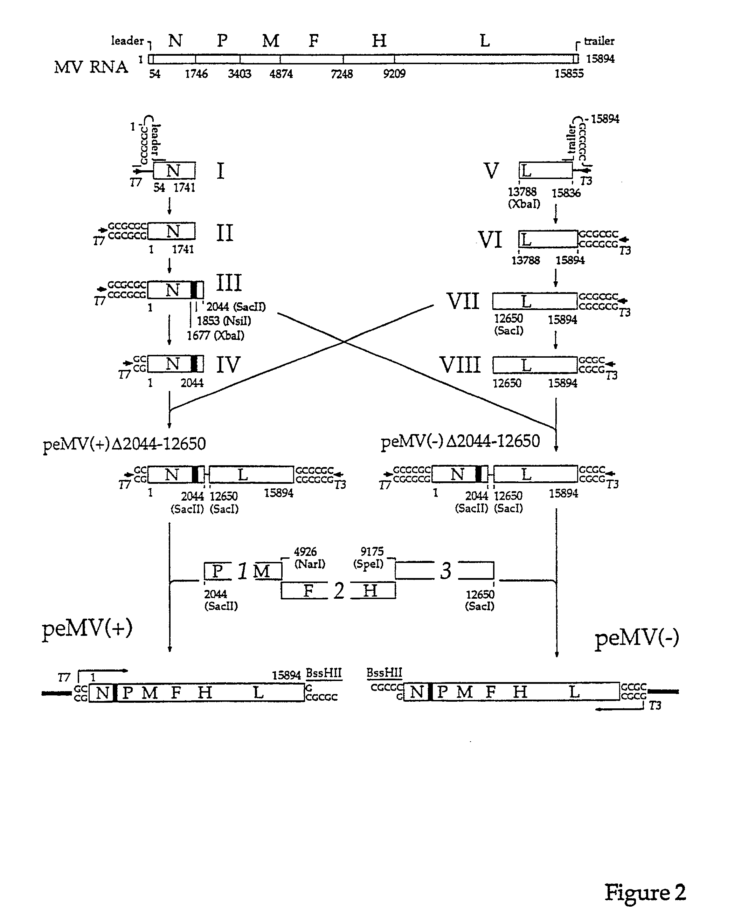

MV is a member of the paramyxovirus family. Its genetic information is encoded on a single strand of RNA ($\tilde{\ }5 \times 10^6$ daltons) with negative polarity. The genome is sequentially transcribed from the 3' terminus to yield 6 major separate ribonucleic acid (RNA) species, each of which encodes one major protein. The genome map is shown in Figure 2 (top), indicating the genes encoding these main products: N (nucleocapsid), P (phosphoprotein), M (matrix), F (fusion), H (hemagglutinin) and L ("large" = polymerase). Several additional RNA and protein species complicate this simple picture, but they are not particularly relevant here.

MV is a major cause of acute febrile illness in infants and young children. At present, several live attenuated MV vaccine strains are used (including Edmonston A and B and their derivatives, the Schwarz and Edmonston-Zagreb strains). Measles vaccine is usually administrated at 15 months of age in developed countries and at 4 to 6 months in developing areas; it has been shown to be highly effective in preventing disease. However, the genetic alterations responsible for attenuation of these vaccine strains remain unknown. The proven safety of measles vaccine, combined with the fact that it is routinely administrated to young children, makes it an ideal "carrier" for heterologous genes. Such a vaccine may prove useful in eliciting immune protection against other pathogenic agents in addition to the protection against the "carrier" virus itself.

The study of the replication cycle of a number of RNA viruses has been greatly facilitated by the availability of DNA clones from which infectious virus can be derived. Initially the Q$\beta$ bacteriophage, then poliovirus and other picornaviruses, and more recently Sindbis, another plus-strand animal RNA virus, were expressed from cloned cDNA (Taniguchi et al, 1978; Racaniello and Baltimore, 1981; Rice et al, 1987). Infectious clones of several plus strand plant RNA viruses (Ahlquist et al, 1984; Dawson et al., 1986; Meshi et al., 1986), an insect plus strand RNA virus (Dasmahapatra et al., 1986), and several viroids and plant satellite RNAs were also described (Cress et al., 1983; Tabler and Sänger, 1985; for references see Simon et al., 1988), and proviral DNA of retroviruses is infectious. Several engineered plus strand RNA viruses have been described for potential use as vectors for immunization purposes, eg. poliovirus, (Evans et al.,1989) and Sindbis (Xiong et al., 1989). However, attempts to obtain infectious virus from cDNA clones of negative-strand RNA viruses have failed so far. This is due to two properties of these viruses: (i) neither genomic nor antigenomic RNAs are infectious, because they do not serve as mRNAs; and (ii) both transcription and replication require ribonucleocapsids, i.e., helical nucleoprotein complexes, containing the genomic RNA and several proteins with structural or enzymatic function. In the case of MV, ribonucleocapsid consists of a $\tilde{\ }$ 16 kb long genomic RNA and at least three proteins, the structural nucleocapsid (N) protein, a polymerase (L for large), and a polymerase cofactor (P for phosphoprotein).

In the past, a variety of DNA viruses and positive-strand RNA viruses have been used as carriers to direct the expression of heterologous genes or gene segments in host cells, with the aim to elicit immune protection against the pathogen from which the heterologous genetic material was derived. In addition to the RNA viruses cited above, the DNA viruses vaccinia virus (Niacin et al., 1982; Weir et al.), cytomegalovirus (Spaete and Mocarski, 1987) and herpes virus (Spaete and Frenkel) should be mentioned here as examples. However, the use of such viruses as carriers suffers from several drawbacks including: (i) potential for inducing severe adverse reactions, as in the case of vaccinia virus; (ii) difficulty in genetic manipulation, specifically limitations on the size of the foreign genetic material which can be introduced into the viral genome without prohibiting packaging in the virion, as in the case of polio virus.

Recently Luytjes et al. (1989) have described a procedure by which a single gene of influenza virus (a segmented negativ-strand RNA virus) can be manipulated recombinant DNA techniques and then amplified in a virus-dependent fashion. In this case, a recombinant RNA containing a foreign coding sequence (chloramphenicol acetyltransferase flanked by the terminal sequences of the viral NS gene) is derived in vitro from plasmid DNA. This RNA is then covered with purified influenza A virus protein and introduced into cells superinfected with helper virus, leading to formation of viral particles which contain not only the 8 genomic RNAs of the helper virus, but also the manipulated RNA. Drawbacks to this approach include: (i) need of helper virus, (ii) need of purified viral proteins, (iii) the fact that influenza virus has a segmented genome, which may result in the loss of introduced heterologous gene elements upon subsequent rounds of replication and (iv) it is unknown whether immunization with a living attenuated influenza strain is able to confer long-lasting immunity.

In order to circumvent the above explained disadvantages, the present invention provides cDNA copies of entire negative-strand RNA virus genomes that are operatively linked to expression control sequences, such as bacteriophage RNA polymerase promoters. In a preferred embodiment said cDNA molecules are inserted into plasmid vectors. A procedure was developed to obtain infectious MV from such vectors. The system was centered around the use of helper cell lines such as cell line IP-3-Ca persistently infected with MV and thus producing all proteins necessary for MV replication, but synthesizing defective proteins M, F and H, and thus not producing any infectious MV particles. The recombinant plasmids described above were introduced into this cell line.

As described below, the system of the present invention allows the introduction of heterologous genes or parts thereof. In this case, negative-strand RNA viruses, such as MV would serve as a carrier. Non-segmented negative-strand RNA viruses are ideally suited as carriers for foreign genes for the following reasons: (i) the genome is nonsegmented; (ii) the genome is sequentially transcribed to produce independent mRNAs for each gene product; (iii) the complete genome is "packaged" into a helical nucleocapsid structure, which results in no stringent constraints upon genome size; (iv) since the genomic RNA is active as template for replication and transcription in an encapsidated form there are probably no general constraints on RNA secondary and tertiary structure; therefore, it can be presumed that a few sequences at the ends of the genome and at the gene boundaries are sufficient to allow recognition by the replication/transcription machinery; (v) the viral proteins are not cleaved from larger precursor proteins. Taken together, these facts permit the insertion of a foreign gene between the genes of the negative-strand RNA virus without interfering with either virus RNA replication and transcription or the production of infectious virions.

Accordingly, it is an object of the invention to provide cDNA molecules containing the entire genomic or antigenomic sequence of negative-strand RNA viruses closely linked to a regulatory region permitting the generation of genomic or antigenomic RNA containing only a few additional nucleotides at both ends.

It is another object of the invention to provide helper cells producing the proteins necessary for encapsidation, transcription and replication of the negative-strand viral genomes and containing said cDNA molecules in the form of a complex with RNA polymerase recognizing the regulatory region.

Another object of the invention is to provide infectious negative-strand RNA virus produced by the above transformed helper cells. These RNA viruses contain a genome assembled by recombinant DNA technology from different parent genomes.

Further objects of the invention are to provide a method for the generation of infectious negative-strand RNA viruses, and vaccines containing at least one type of said infectious negative-strand RNA viruses.

Negative-strand RNA viruses which are preferred as carriers for the present invention are measles and mumps viruses.

Examples of preferred heterologous genes or gene fragments inserted into the genomes of the virus carriers of the present invention include the following:

1) Rotavirus: VP1 through VP7, alone or in combination, from serotypes of rotavirus pathogenic for humans.

2) Hepatitis A Virus: VP1 through VP4, preferentially VP1 or a genomic fragment encoding at least amino acids 13-24.

3) Hepatitis B Virus: HBsAg or a genomic fragment encoding amino acids 176-311.

4) Respiratory Syncytial Virus: F and/or G protein.

5) Plasmodium falciparum: Gene fragments encoding $(NANP)^N$ or $(NANP)^N NVDP(NANP)^N$.

6) HIV GP 120, GP 41, P24 and fragments thereof, alone or in combinations.

7) Bordetella pertussis: A gene encoding pertussis toxin (PT) lacking enzymatic activity.

The above objects are met by providing the embodiments characterized in the claims. Further objects and advantages of the present invention will be apparent from the following description.

In one embodiment, the present invention relates to the production of attenuated negative-strand RNA viruses derived by specific mutation of specific genes.

In another embodiment, the invention relates specifically to a measles virus strain derived from Edmonston A or B (or one of their derivatives Schwarz or Edmonston-Zagreb) vaccine strain into which was introduced heterologous gene material (entire genes or parts thereof) encoding an antigen or antigens, which give rise to a protective immune response upon administration to humans.

In another embodiment, the invention relates specifically to a mumps virus strain derived from the Rubini, Jeryl Lynn, or Urabe vaccine strains, into which was introduced heterologous gene material.

In another embodiment, the present invention relates to a measles or mumps vaccine comprising the above described virions in an amount sufficient to elicit a protective immune response to the carrier strain of measles or mumps, to the heterologous pathogen, or to both.

In another embodiment, the present invention relates to a method of preparing a live attenuated vaccine against negative-strand RNA viruses, specifically MV or mumps virus, comprising the steps of:

(i) cloning full length genomic cDNA copies by assembly of cDNA clones derived from mono- and polycistronic viral messenger RNA and chemically synthesized oligonucleotides (or possibly direct cloning of full lenth genomic cDNA from genomic RNA with the use of oligonucleotides) in vectors containing phage RNA polymerase promoters, in such a way as to allow enzymatic synthesis of viral genomic or antigenomic RNA containing only a few additional nucleotides at both ends;

(ii) exchanging a DNA segment of the cloned genomic sequence with a homologous DNA segment containing known mutations (alternatively, the mutationally altered segment can be introduced already in step (i));

(iii) cloning the recombinant DNA vector in Escherichia coli, verifying the introduced mutated sequence by partial sequence determination and linearizing by cleavage with a restriction endonuclease closely downstream of the viral genomic insert sequence;

(iv) introducing the DNA as a complex with phage T3 or T7 RNA polymerase into competent cells (eg. IP-3-Ca) producing the viral proteins required for genome RNA packaging, transcription and replication;

(v) recovering the infectious virus particles excreted from the cells mentioned above and amplifying them in cells permissive for normal viral replication; and

(vi) formulating the virus produced in (v) into a galenic formulation appropriate for administration to humans.

In another embodiment, the present invention relates to a method of producing a modified live attenuated negative-strand RNA virus, specifically MV or mumps virus, carrying parts of genes of heterologous pathogens encoding epitopes able to elicit a protective immune response to said heterologous pathogens, comprising the steps of

(i) cloning full length viral genomic cDNA copies as described in the previous embodiment;

(ii) inserting short segments encoding epitopes of heterologous pathogens into the coding sequences of the viral genes for structural proteins (particularly envelope proteins) in a manner such as to maintain the reading frame;

(iii) cloning the recombinant DNA vector in Escherichia coli, (as in iii above);

(iv) introducing the DNA as a complex into comptent cells (as in iv above);

(v) recovering the infeotious virus (as in v above); and

(vi) formulating the virus produced (as in vi above).

In another embodiment the present invention relates to a method of producing a hybrid live attenuated negative-strand RNA virus, in which a structural protein is entirely or partly substituted by the corresponding structural protein or protein domain of a closely related virus comprising the steps of

(i) cloning full length viral genomic cDNA copies (as in i above);

(ii) replacing the coding region of a structural gene partly or entirely by the corresponding coding region of the homologous gene of a closely related virus;

(iii) cloning the recombinant DNA vector in Escherichia coli (as in iii above);

(iv) introducing the DNA as a complex into competent cells (as in iv above);

(v) recovering the infectious virus (as in v above); and

(vi) formulating the virus produced (as in vi above).

In another embodiment, the present invention relates to a method of producing an attenuated negative-strand RNA virus producing an additional protein of an unrelated pathogen able to elicit a protective immune response, comprising the steps of

(i) cloning full length viral genomic cDNA copies (as in i above);

(ii) inserting the region encoding the protein of the unrelated pathogen, flanked by intergenic sequences typical for the virus used, precisely between two viral genes, in a manner such that its expression is governed in the same way as the genes of the carrier virus;

(iii) cloning the recombinant DNA vector in Escherichia coli (as in iii above);

(iv) introducing the DNA as a complex into competent cells (as in iv above);

(v) recovering the infectious virus (as in v above); and

(vi) formulating the virus produced (as in vi above).

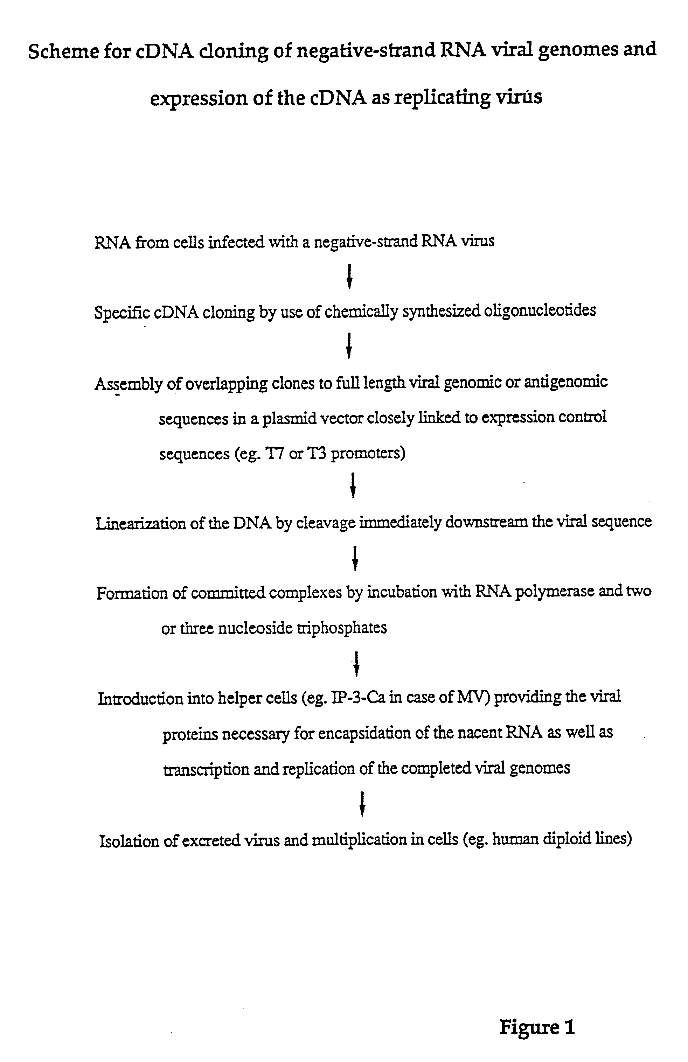

Figure 1: Summary of the process used to obtain infectious negative-strand RNA viruses from cloned cDNA.

Figure 2: Scheme for the construction of vectors able to produce full length transcripts of genomic negative-strand MV RNA by incubation with T3 RNA polymerase (linearized plasmid peMV(-)) or full length transcripts of antigenomic positive-strand MV RNA by incubation with T7 polymerase (linearized plasmid peMV(+)). A detailed explanation of the symbols and the construction steps is provided in the text to example 1.

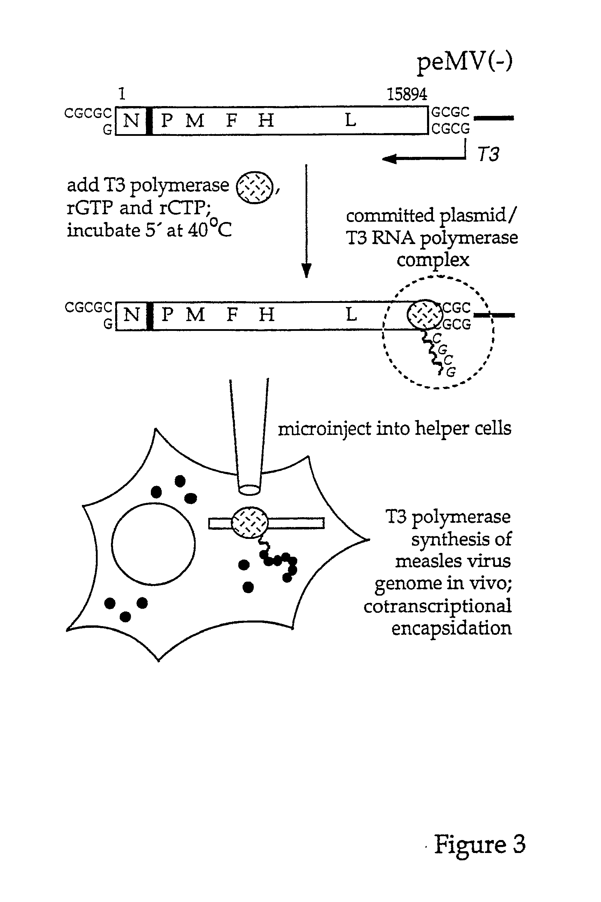

Figure 3: Infectious measles virus from cloned cDNA: expression methodology. Plasmid peMV(-), shown after linearization with BssHII, and strategy for synthesis of MV genomic (minus-strand) RNA in helper IP-3-Ca cells. Symbols are as in Figure 2, with the following additions: T3 RNA polymerase is represented as a stippled oval, nascent RNA chains by wavy lines, N protein by black dots.

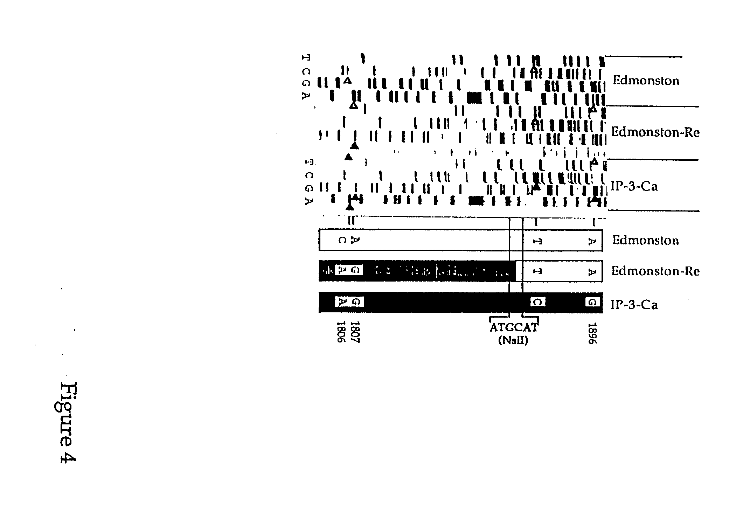

Figure 4: Analytical characterization of a reconstructed virus: Direct sequence determination of part of the Edmonston, Edmonston-Re and IP-3-Ca P genes on cDNAs amplified by polymerase chain reaction. Nucleotides specific for the Edmonston sequence are highlighted with white triangles, those specific for the IP-3-Ca sequence with black triangles. To the right of the sequencing ladders the P gene regions corresponding to the analysed DNA regions are schematically shown. White areas represent Edmonston or Edmonston-derived sequences, black areas IP-3-Ca or IP-3-Ca-derived sequences. The nucleotides highlighted in the sequencing gel are indicated in the corresponding areas. The NsiI site used for construction of the Edmonston-Re genome is indicated on the right, where the position of the relevant nucleotides in the MV antigenome is also indicated. Note that the P gene of the Edmonston virus substrain used in this study differs from the one sequenced by Bellini et al. (1985), in that it contains an A residue instead of a G at position 1807. The P gene coding region begins with the ATG at positions 1808-1810.

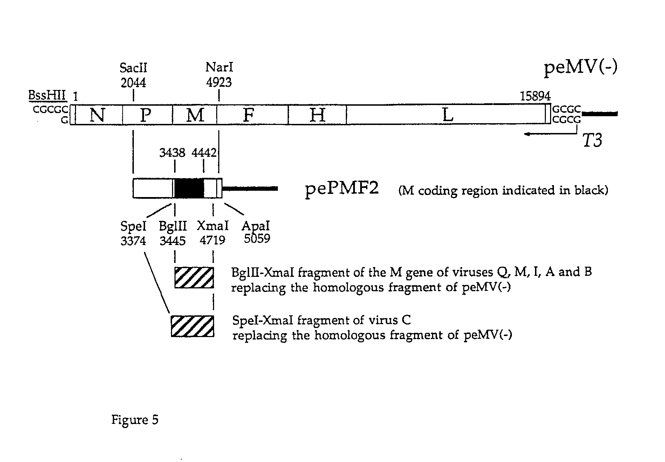

Figure 5: Plasmids used for the functional analysis of M genes. On the top, plasmid peMV(-) (see Figures 2 and 3) is represented. The SacII and NarI restriction sites used for the M gene exchange from the intermediate plasmid pePMF2 (centre) are indicated. Nucleotide numbering is according to Cattaneo et al. (1989b; EMBL GenBank accession number X 16565-9). The black box indicates the M coding region and the numbers its boundaries. On the bottom, the BglII-XmaI fragment from SSPE viruses M, I, A and B and from lytic virus Q, and the SpeI-XmaI fragment from virus C which have been transferred first into pePMF2 and then into peMV(-), are indicated.

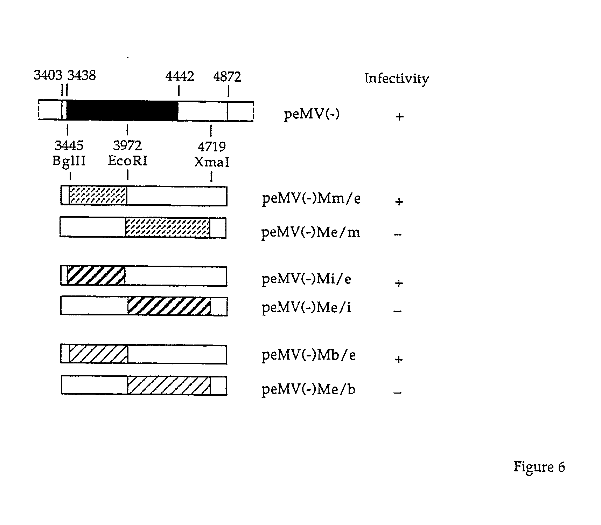

Figure 6: Structure of the chimeric M genes (left), and results of the functional analysis (right). On the top left, the M coding region is indicated as a black box. Below this, the parts of the genes transferred to peMV(-) from cDNAs of cases M, I or B are represented as hatched boxes. For transfer, the BglII, EcoRI and the XmaI sites shown were used. The BglII-EcoRI fragment corresponds to the region encoding amino acids 4-180; the EcoRI-XmaI fragment contains the region encoding amino acids 179-335. Note that an additional EcoRI site at position 4383 exists, but is not shown. For this reason, partial EcoRI digestion conditions had to be used to prepare the EcoRI-XmaI fragment. On the right, infectivity, indicated as (+), means production of infectious virus, as monitored by complete cell lysis within six days from beginning of infection, and reproducibly observed in two different microinjection experiments.

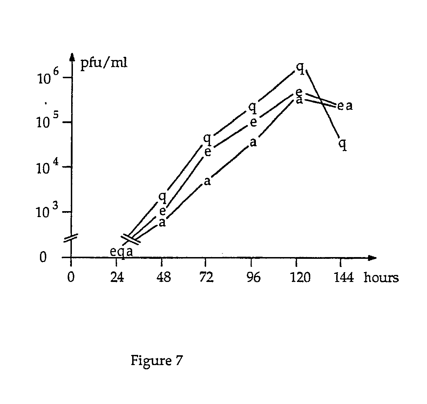

Figure 7: Growth curve of measles viruses carrying different M genes. Viruses recovered from the supernatant of microinjected IP-3-Ca cells were subjected to one cycle of plaque purification and used to infect in parallel Vero cells. At various times after infection, as indicated on the bottom, supernatants were collected from the cultures, stored at -70° C, and were later used for viral plaque assay. Titers of viruses obtained from plasmids peMV(-), peMV(-)Mq and peMV(-)Ma are indicated by the lower case letters e, q and a, respectively.

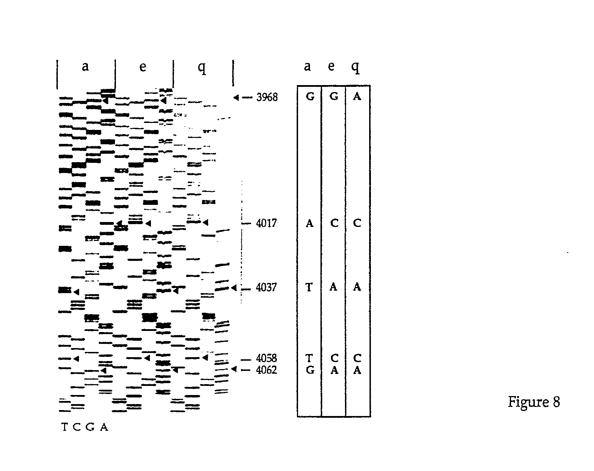

Figure 8: Direct sequence determination of the part of the M genes of viruses obtained from plasmids peMV(-) [indicated as "e"], peMV(-)Mq ["q"] and peMV(-)Ma ["a"]. Total RNA from cells infected with these viruses was reverse transcribed, the cDNA amplified by PCR, and sequenced essentially as described in Materials and Methods. Left: autoradiogram of the sequencing gel. Nucleotides showing differences in the sequences of the three viruses are indicated with black triangles. Right: schematic drawing of the M gene sequences corresponding to the analyzed DNA regions. Nucleotide differences between the sequences are indicated, as well as the positions at which these differences occur.

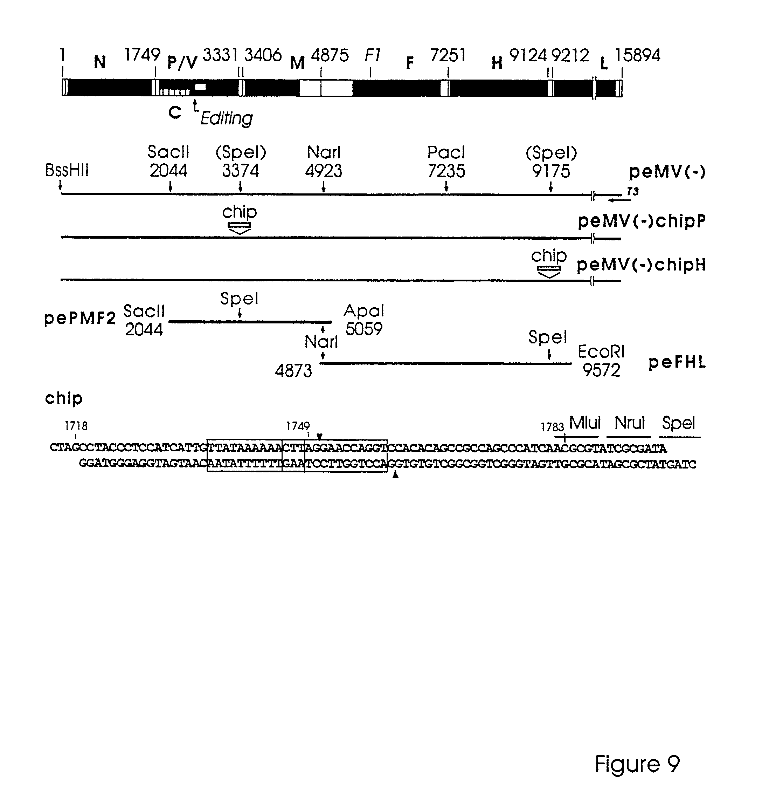

Figure 9: Overview of plasmids and DNA fragments used for the construction of plasmids containing the framework of a seventh transcription unit. Top: representation of the MV genome drawn colinear to the MV cDNA plasmids shown below. The six transcription units are labelled with the letters indicating their encoded proteins. The main protein reading frames are depicted in black, those accessed by alternative protein initiation (C) and by editing (V) by small hatched and white areas, respectively. Nucleotide numbers (Cattaneo et al., 1989b) mark the termini of the genome and the starting nucleotides of most transcription units and the termination codons of the P and H reading frames. Middle: schematic representation of the plasmid end products (peMV(-)chipP, peMV(-)chipH) and plasmid intermediates, relevant for the constructions. The synthetic chip (bottom) was first inserted in the unique SpeI site of plasmids pePMF2 and peFHL, to yield pePMF2chip and peFHLchip, (not represented). Appropriate SacII-NarI fragments and NarI-SpeI fragments then replaced the homologous region in peMV(-), as described in the text. Relevant restriction sites, unique for the plasmids represented, are indicated (the Spe I sites in brackets are not unique for peMV(-)). Only the MV cDNA inserts are drawn; the bluescript vector sequences linking the termini are omitted. Bottom: nucleotide sequence of the synthetic chip used for insertion. All nucleotides between 1718 and 1783 agree precisely with the N/P gene boundary of the MV genome. The boxed nucleotides, embracing the entire region showing discernable homology to other MV gene boundaries, are presumably sufficient to specify transcription stop, polyadenylation and restart. The joining points of the synthetic oligonucleotides are indicated by arrowheads.

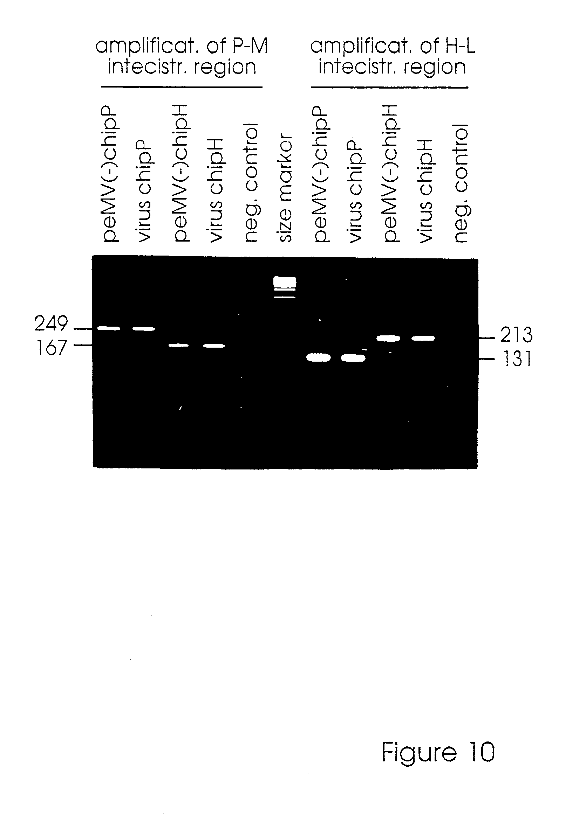

Figure 10: Agarose gel electrophoresis of PCR amplification products. Templates for PCR amplification spanning the intercistronic regions P/M and H/L, respectively, were preparations of total RNA from cells infected with reconstructed viruses, retrotranscribed into cDNA (virus chipP and virus chipH). As controls and size markers, template DNA of the plasmids used to give rise to the reconstructed viruses was employed. Electrophoresis was through a 2.5% agarose gel stained with ethidium bromide.

A summary of the steps necessary to create negative-strand RNA viruses from cloned cDNA is provided in Figure 1. The invention is illustrated by the following examples:

## Example 1: Plasmids allowing synthesis of Edmonston strain genomic RNA

The reference MV vaccine strain Edmonston was chose as the source of the RNAs needed for genome reconstruction. Full length cDNA copies of mono- and polycistronic mRNAs were cloned according to Schmid et al. (1987), and assembled with synthetic oligonucleotides corresponding to the MV genomic ends to reconstitute the 15894 nucleotide MV genome. Obvious concerns were the documented heterogeneity of the MV RNA genome and the occurrence of editing, giving rise to mRNAs different from the cognate MV genomic sequence (Cattaneo et al., 1989a). Therefore, the intactness of all protein coding regions of the clones used for genome reconstruction was verified by *in vitro* translation of synthetic transcripts (Cattaneo et al., 1988b).

Figure 2 shows the strategy used for the construction of DNA copies of the MV genome in vectors allowing production of transcripts corresponding to the MV genome or antigenome. On the top, the MV RNA antigenome with its six genes, the leader and the trailer sequences is shown. The first nucleotide of each gene is indicated using the system of Cattaneo et al. (1989b; EMBL/Genbank accession number X 16565-9). Under the N and the L genes, plasmids encompassing the ends of the genome are described (I to IV and V to VIII, respectively). The final products, peMV(+) and peMV(-), are drawn (not to scale) on the bottom left and right, respectively. Some intermediate constructions are not shown, and vector sequences (pBluescript + KS) are indicated by black bars only in the final products. A complete description of the construction scheme can be found in Eschle (1988). Methods were basically as in Maniatis et al. (1982).

Steps I-II and V-VI: joining of the T7 and T3 promoters with the MV genomic sequence, and elimination of the polylinker sequence, via introduction of the MV leader and trailer sequences into clones derived from mRNAs. Oligonucleotides (105 and 103 nucleotides long) encompassing the leader and the trailer sequence respectively, BssHII sites (GCGCGC), and about 20 nucleotides on either side corresponding to flanking plasmid promoter and MV N gene or L gene sequences, respectively, were synthesized. These oligonucleotides were hybridized with single-stranded vector DNAs obtained by superinfection of cells containing plasmid I or V with phage M13. After elongation *in vitro* and ligation, double-stranded covalently closed DNA was isolated by gel electrophoresis and used to transfect competent *E. coli* cells; clones hybridizing to the corresponding end-labelled oligonucleotides were selected. In the case of plasmid II, the 60 bases gap between the T7 promoter and the start of the N gene was bridged by the oligonucleotide containing the leader sequence. In the case of the other genomic end, only a clone missing some nucleotides of the trailer region was obtained. This clone was then completed by inserting synthetic DNA consisting of four predominantly complementary, overlapping oligonucleotides.

Step II-III: insertion of a genetic tag at the N-P junction. A 176 bp long XbaI-NsiI fragment from ε cDNA containing the N-P intercistronic region derived from a IP-3-Ca mRNA library (black box; Cattaneo et al., 1988a), and a contiguous MV Edmonston P gene NsiI-SacII fragment, were ligated into vector II.

Step VI-VII: enlargement of the L gene region.

Steps III-IV and VII-VIII: elimination of BssHII sites. Plasmids III and VII were cleaved with BssHII and G and C bases eliminated from the 3' ends using T4 DNA polymerase in the presence of only dATP and dTTP. 5' overhanging nucleotides were then trimmed using mung bean nuclease. For plasmid IV, clones with 1, 2 or 4 nucleotides remaining were obtained and tested for transcription with T7 RNA polymerase. Only clones with 2 or 4 nucleotides yielded acceptable amounts of RNA (25% as compared to the original T7 GGG initiation site), whereas a clone containing only 1 additional nucleotide (C) yielded almost no RNA. For plasmid VIII, only clones with 4 nucleotides remaining were obtained.

peMV(+)Δ2044-12650 and peMV(-)Δ2044-12650: vectors containing both MV genomic ends. The trailer sequence of peMV(+)Δ2044-12650 is followed by a unique BssHII site; this plasmid was obtained by ligation of vector IV with the insert of vector VII and a small unrelated SacI-SacII fragment. The leader sequence of peMV(-)Δ2044-12650 is preceded by a unique BssHII site; this plasmid was obtained by ligation of vector III with the insert of vector VIII and a small SacI-SacII fragment.

Fragments 1, 2 and 3: fragment 1 was obtained from a reconstituted plasmid containing a cDNA insert encompassing contiguous sequences of most of the P, the whole M, and part of the F gene. In the construction of this plasmid, a cDNA covering the M-F intercistronic region from an MV Edmonston strain genomic library (Cattaneo et al., 1987) was used. Fragments 2 and 3 were excised from plasmids with inserts corresponding to bicistronic F-H and H-L mRNAs, respectively.

Plasmids peMV(+) and peMV(-) (not to scale): these plasmids were obtained by one-step ligations of fragments 1, 2 and 3 with, respectively, vectors peMV(+)Δ2044-12650 or peMV(-)Δ2044-12650, linearized by SacII-SacI cleavage. Plasmids are shown with vector sequences (black bars), such as they are used for *in vitro* transcription after linearization with BssHII. The T7 promoter in peMV(+) and the T3 promoter in peMV(-) are shown, as are the expected points of initiation of transcription, situated in sequences flanking the MV inserts.

In these artificial MV DNA genomes a genetic tag (black box) was introduced: a short fragment of cDNA obtained from the SSPE-derived defective IP-3-Ca-MV (persisting in IP-3-Ca cells) was inserted rather than the corresponding piece from the Edmonston sequence. This fragment contains three nucleotides differing from the Edmonston genome: an A residue instead of a G in the 3' untranslated region of the N gene, and an A followed by a G (instead of a C and an A, respectively) in the 5' untranslated region of the P gene.

The final products of the reconstruction are the two ~ 19 kilobase (kb) long plasmids peMV(+) and peMV(-), shown at the bottom of Figure 2. From peMV(-), full length minus strand genomic RNAs can be obtained by T3 polymerase transcription of templates linearized with BssHII. These RNAs are expected to contain four additional nucleotides (GCGC) at the 5' end and five additional nucleotides (GCGCG) at the 3' end. Conversely, T7 polymerase transcription of BssHII linearized peMV(+) DNA yields full length plus strand antigenomic RNA with two additional nucleotides (GC) at the 5' end and five (GCGCG) at the 3' end. Transcripts of the MV insert of peMV(-) and peMV(+) were analysed on agarose gels after denaturation. Transcripts of up to ~ 16 kb in length were obtained when the plasmids were linearized with BssHII, and transcripts of larger sizes when the plasmids were linearized by a restriction enzyme cutting downstream of the MV sequences (data not shown). This analysis proved that no strong stop signals for T3 and T7 polymerases are present in the MV genomic and antigenomic sequences.

Example 2: Virus can be reproducibly recovered from the supernatant of helper cells after microinjection of committed plasmid DNA/phage RNA polymerase complexes

To start productive infection, synthetic MV RNA genomes have to be complexed with the N, P and L proteins necessary for transcription and replication. In principle, this might possibly be achieved by reconstitution of ribonucleocapsids *in vitro*. However, it appeared more promising to introduce MV RNA into cells producing the proteins necessary for MV replication in high quantities and in the appropriate ratio. The cell line IP-3-Ca, obtained by cocultivation of brain tissue from an SSPE patient with cells permissive for MV replication (Burnstein et al., 1974), was selected for our studies because it supports efficient propagation of the resident MV genome but does not release infectious virus, as a consequence of defects in the genes for the envelope matrix (M), fusion (F) and hemagglutinin (H) proteins (Sheppard et al., 1986; Cattaneo et al., 1989b).

Experiments involving transfection, lipofection and electroporation of IP-3-Ca cells with genomic RNA extracted from ribonucleocapsids, and electroporation of synthetic genomic RNA produced from peMV(-) and peMV(+) yielded negative results, in spite of the fact that productive transfection of IP-3-Ca cells with Edmonston-genome containing ribonucleocapsids could be achieved. The reason for these failures might reside in the difficulty of introducing an RNA of almost 16 kb into the helper cells in an undegraded form. Alternatively, if the MV genomic RNA encapsidation site, residing close to the 5' terminus, is accessible only when this RNA is being synthesized, and covered up in longer RNA chains, full length RNA introduced in IP-3-Ca cells would fail to be correctly encapsidated.

To generate nascent MV genomic RNAs directly in IP-3-Ca cells, we introduced into these cells the linearized plasmids peMV(-) or peMV(+) together with the corresponding phage RNA polymerases. We reasoned that transcription would be favoured if committed replication complexes would be preformed *in vitro*, rather than after microinjection of a mixture of enzyme and template. To this purpose we incubated plasmid DNAs and RNA polymerases with GTP and CTP but without ATP and UTP; under these conditions polymerases should initiate transcription but stop synthesis after few nucleotides (Figure 3, center, shows a representation of a committed peMV(-)/T3 RNA polymerase complex, on which an RNA chain of four nucleotides has formed). Committed complexes were microinjected into the cytoplasm of IP-3-Ca cells, after ascertaining that microinjection did not cause shearing of plasmid DNA. Three events were expected: first, that at least some committed complexes would resume RNA synthesis in the presence of the intracellular nucleotide pool; second, that the MV N protein available in IP-3-Ca cells could co-transcriptionally encapsidate the nascent MV RNA genomes (Figure 3, bottom); and third, that these events would allow the generation of functional MV ribonucleoprotein helices with which the P and L proteins responsible for transcription and replication have associated.

Table I  Plaques obtained on Vero cells from the supernatants of microinjected IP-3-Ca cells [a]

| Experiment number | | 1 | 2 | 3 | 4 | 5 | 6 |
|---|---|---|---|---|---|---|---|
| Polarity of RNA produced | | (-) | (+) | (-) | (-) | (-) | (+) |
| supernatant from day | 5 | 1 [b] | 3 | 3 | 1 | 5 | 11 |
| | 7 | 1 | 6 | 4 | 7 | 7 | 18 |
| | 12 | 1 | 5 | 3 | 4 | 10 | 5 |
| | 14 | 2 | 4 | 3 | 3 | 9 | 4 |
| Negative controls [c] | | | | | | | |
| supernatant from day | 5 | nd | nd | 0, 0 | nd | 0 | 0 |
| | 7 | nd | nd | 0, 0 | nd | 0 | 0 |
| | 12 | nd | nd | 0, 0 | nd | 0 | 0 |
| | 14 | nd | nd | 0, 0 | nd | 0 | 0 |

[a]  Results are expressed as number of plaques per 0.5 ml supernatant of IP-3-Ca cells.

[b]  In this experiment supernatant of IP-3-Ca cells taken two days after infection was found to yield no plaques.

[c]  Negative controls were the following: minus strand plasmid alone and T3 RNA polymerase alone in experiment 3. Minus strand committed complex producing incomplete transcripts in experiment 5. Plus strand committed complex producing incomplete transcripts in experiment 6. The plasmids used in the negative controls of experiments 5 and 6 were linearized with NarI (the unique NarI site in the MV genome is situated at position 4926, Figure 1).

nd  Not determined.

That this was indeed the case was suggested by the reproducible isolation of infectious virus from supernatants of IP-3-Ca cells into which committed complexes had been microinjected. The results are shown in table 1. Supernatants of IP-3-Ca cells collected 5, 7, 12 or 14 days after microinjection with committed complexes producing genomic (experiments 1, 3, 4 and 5) or antigenomic (experiments 2 and 6) transcripts yielded a small number of plaques in all experiments. Supernatants from negative controls, in which only plasmid DNA or only phage RNA polymerase were injected, were negative (experiment 3, negative controls), and the same was true for experiments with committed complexes in which the plasmid DNA was cut within the MV insert (experiments 5 and 6, negative controls).

Example 3. Reconstituted virus propagates lytically and contains the genetic tag introduced in the cDNA

To prove conclusively that MV can be expressed from DNA copies of MV RNA genomes we had to show that the infectious agent forming plaques on Vero cells propagated lytically, unlike the strictly cell-associated MV genome present in IP-3-Ca cells, and that the three point mutations introduced in the reconstituted Edmonston DNA were present in the genome of the plaque forming agent. A mass infection was started by the addition of 0.5 ml supernatant (containing about 5 PFUs) from IP-3-Ca cells microinjected with peMV(-)/T3 RNA polymerase complexes to $3 \times 10^5$ Vero cells. After 6 days these cells lysed, and one third of the supernatant was used to infect $2 \times 10^6$ Vero cells. Formation of small syncytia was noted 24 hr after this infection, and RNA was extracted after 60 hr, when the majority of the cells were engaged in large syncytia. These observations indicate the rapid propagation of a lytic, rather than of a slowly progressing cell-associated virus.

The sequences of the relevant parts of the N and P genes of the reconstituted virus (Edmonston-Re), and of the corresponding Edmonston and IP-3-Ca genes as controls, were obtained after reverse transcription of their mRNAs, using primers specific for the N and P genes. These N and P cDNAs were amplified by polymerase chain reactions (Saiki et al., 1988) and the resulting DNAs were sequenced directly. The results are shown in Figure 4 for the relevant part of the P genes. Nucleotides specific for the Edmonston sequence are highlighted with white triangles, those specific for the IP-3-Ca sequence with black triangles. To the right of the sequencing ladders the P gene regions corresponding to the analyzed DNA regions are schematically shown. White areas represent Edmonston or Edmonston-derived sequences, black areas IP-3-Ca or IP-3-Ca-derived sequences. The nucleotides highlighted in the sequencing gel are indicated in the corresponding areas. The NsiI site used for construction of the Edmonston-Re genome is indicated on the right, where the position of the relevant nucleotides in the MV antigenome is also indicated. From these data it is clear that the Edmonston-Re sequence is identical to the Edmonston sequence, with the exception of two bases in the untranslated region (an A at position 1806 and a G at position 1807), which conform to the IP-3-Ca sequence used to tag this part of the genome. Analysis of the relevant part of the N gene of Edmonston-Re confirmed that its sequence differs from the one of Edmonston as predicted only by a G to A substitution at position 1703 (data not shown).

Example 4: Plasmids containing genomic MV cDNA are suitable for genetic manipulations aimed to assay the functionality of altered genes.

It was important to test whether our plasmids able to give rise to infectious MV readily allow genetic manipulations involving replacement of long DNA segments. As a practical application we used the coding area of naturally mutated MV M genes to replace the homologous area of the resident M gene originating from the MV Edmonston strain. This allowed us to determine whether the M proteins encoded by the mutated M genes were functionally competent: only plasmids containing in their reconstituted genomic MV cDNA an M gene able to encode functional protein can be expected to give rise to replicating virus.

The mutated M gene fragments to be tested had been cloned from cases of persistent MV infections (Cattaneo et al., 1989b) leading to the lethal syndromes subacute sclerosing panencephalitis (SSPE) and measles inclusion body encephalitis (MIBE). The functional characterization of these M genes was of considerable interest, since it has been hypothesized that a prerequisite for the development of these syndromes was either a complete lack or a functional inactivation of the MV M gene products.

Four individual mutated M genes were tested: two had been cloned from persistently MV-infected cell lines MF and IP-3-Ca, derived from two SSPE cases, two others directly from autopsy material of SSPE cases A and B (designated as M, I, A and B, respectively). As positive control, an M gene cloned from the lytic strain Toyoshima CAM-RB (indicated as Q) was used. As negative control an M gene cloned from MIBE case C (indicated as C) was used, in which the start, the stop and dozens of other codons are altered as the result of a hypermutation event.

The exchange was carried out in two steps (Fig. 5). In a first step, BglII-XmaI fragments of clones of the M gene of viruses Q, M, I, A and B were exchanged with the homologous fragment in the short plasmid pePMF2; it was necessary to use a plasmid containing only a short MV cDNA insert spanning from the P over the M gene into the F gene, in which the restriction sites used for the exchange were unique. For the M gene clone of virus C, in which a mutation has led to the elimination of the BgIII site, an SpeI site located at the 3' end of the P gene was used to generate an SpeI-XmaI fragment which was analogously exchanged. Thus, a total of six derivatives of pePMF2 were generated. In a second step, plasmid peMV(-) was cleaved with SacII and NarI and the 2879 bp long SacII-NarI fragment was substituted by the homologous fragment of plasmid pePMF2 derivatives. The final products are plasmids peMV(-)Mq, peMV(-)Mm, peMV(-)Mi, peMV(-)Ma, peMV(-)Mb and peMV(-)Mc, which differ from the Edmonston genomic cDNA only in the M genes.

Note that the replacing DNA fragments covered the entire coding region of the mutated M genes, except for the the first three amino acids, which are conserved in all strains analyzed. The deviations of the predicted amino acid sequence (as deduced from the sequence determination of the respective cDNAs) from the consensus M protein sequence (Cattaneo et al., 1988b) are represented in Table 2.

The linearized MV genomic plasmids containing the different M genes were associated with T3 RNA polymerase ("committed complexes", as described in example 2) and microinjected into the cytoplasm of helper cells; the supernatants were then analyzed for infectivity. The results of this analysis are summarized in Table 3.

Table 2  Amino acid differences among
functional and non-functional M genes.

| Pos. | Cons. | Case | | | | | |
|---|---|---|---|---|---|---|---|
| | | E | Q | A | M | I | B |
| 5 | Y | . | . | . | . | H | . |
| 11 | A | . | . | . | . | E | . |
| 35 | V | . | . | . | A | . | . |
| 38 | I | . | . | . | . | T | . |
| 60 | E | . | . | G | . | . | . |
| 64 | P | . | S | . | . | . | . |
| 68 | P | . | . | L | . | . | . |
| 83 | S | . | . | . | F | . | . |
| 89 | E | K | K | . | . | . | . |
| 104 | T | . | . | . | . | I | . |
| 117 | T | . | . | . | . | P | . |
| 119 | L | . | . | . | . | . | P |
| 123 | T | . | . | . | . | . | I |
| 129 | L | . | . | . | P | . | . |
| 142 | N | S | . | . | . | . | . |
| 146 | L | . | . | . | . | . | P |
| 155 | F | . | . | . | . | . | L |
| 159 | Y | . | . | . | . | . | H |
| 166 | S | . | . | P | . | . | . |
| 177 | M | . | I | . | . | . | . |
| 181 | R | . | . | . | . | K | . |
| 189 | N | . | . | . | . | K | . |
| 190 | L | . | . | P | . | . | . |
| 194 | L | . | . | I | . | . | . |
| 197 | D | . | . | . | . | H | . |
| 202 | P | . | . | . | . | . | H |
| 209 | A | T | T | . | T | . | . |
| 214 | E | . | . | . | K | . | . |
| 221 | I | . | . | . | T | . | . |
| 228 | K | . | N | . | . | . | . |
| 249 | A | . | . | . | . | . | S |
| 267 | S | . | . | . | . | N | . |
| 284 | L | . | . | P | . | . | . |
| 285 | M | . | I | . | . | . | . |
| 291 | L | . | . | P | . | P | . |
| 294 | L | . | . | S | . | . | . |
| 299 | R | . | . | . | I | . | . |
| 302 | I | . | . | . | . | T | . |
| 303 | V | . | . | . | . | I | . |
| 316 | E | . | . | . | . | . | K |
| 325 | I | . | . | M | . | . | . |

Table 2

Table 3  Functional test of matrix genes of persistent measles viruses

| | | M protein expression by original virus | Production of virus after M gene exchange | | | | | | | | | | | |
| | | | Experiment 1 | | | | | | Experiment 2 | | | | | |
| | | | Infection, day | | | Plaques, day | | | Infection, day | | | Plaques, day | | |
| | | | 5 | 7 | 12 | 5 | 7 | 12 | 5 | 7 | 12 | 5 | 7 | 12 |
| Lytic strains | | | | | | | | | | | | | | |
| Edmonston | E | normal | +a | + | + | 2 | 2 | 3 | + | + | + | 5 | 2 | 4 |
| Toyoshima CAM-RB | Q | normal | + | + | + | 7 | 1 | 1 | + | + | + | 4 | 3 | 5 |
| Persistent strains | | | | | | | | | | | | | | |
| MF | M | not detectable (defective transcription) | – | – | – | 0 | 0 | 0 | – | – | – | 0 | 0 | 0 |
| IP-3-Ca | I | protein unstable ($t_{1/2}$=70 min) | – | – | – | 0 | 0 | 0 | – | – | – | 0 | 0 | 0 |
| A | A | low in brain cells | – | + | – | 1 | 0 | 0 | + | + | + | 1 | 0 | 0 |
| B | B | not detectable in brain cells | – | – | – | 0 | 0 | 0 | – | – | – | 0 | 0 | 0 |
| C | C | start and stop codon mutated | – | – | – | 0 | 0 | 0 | – | – | – | 0 | 0 | 0 |

a (+) means complete cell lysis after six days

As expected, the M genes of lytic viruses Q and E were both compatible with production of replicating recombinant virus. In contrast, three of the four SSPE M genes, and the MIBE M gene used as negative control were not able to functionally replace the resident M gene. Interestingly, the M gene of SSPE case A proved to be competent for production of virus, as reproducibly observed in two different microinjection experiments and with two different assays for infectivity: direct observation of cytopathic effects, and plaque assay. Thus, contrary to the hypothesis mentioned above, inactivation of the M gene function does not appear to be a strict requirement for the development of SSPE.

To narrow down the region containing deleterious mutations in the M genes of the SSPE MV genomes M, I and B, we constructed infectious cDNA plasmids in which either the region encoding the amino- or the carboxyl-terminal half of the resident, functional M protein was replaced by the corresponding half of the M gene of viruses M, I or B (Fig. 6, left). The three plasmids specifying the amino-terminal half of the SSPE M proteins (peMV(-)Mm/e, peMV(-)Mi/e and peMV(-)Mb/e) were able to generate infectious MV, in contrast to the plasmids encoding the carboxyl-terminal half of the SSPE M proteins. The encircled area of Table 2 highlights the aminoacid replacements among which those giving rise to nonfunctional M proteins must be located.

Example 5: Characterization of the virus originating from MV genomic cDNA plasmids carrying an exchanged M gene or M gene fragment.

To obtain further insights into the biological characteristics of the virus containing the SSPE-derived M gene, produced from the plasmid peMV(-)Ma (virus [a]), as compared with that of viruses produced from plasmids peMV(-)Mq (virus [q]) and peMV(-) (virus [e]), parallel infections of Vero cell monolayers were started using viruses recovered from single plaques. Direct microscopic observation of these infections revealed similar cytopathic effects; large syncytia were formed at day 3 and complete lysis occurred at day 6 post infection. At 24 hours intervals, the supernatants of the infected Vero cells were collected and viral titers determined. As shown in Fig. 7, the growth curves were very similar for all three viruses. Characterization of the viruses carrying the chimeric M genes showed that they reached titers similar to viruses [a], [e] and [q] (results not shown).

To ascertain whether virus [a] was indeed derived from plasmid peMV(-)Ma, the coding region of the M gene of this virus was sequenced after reverse transcription of the RNA recovered from infected cells, followed by polymerase chain reaction amplification. In parallel, RNA from cells infected by viruses [e] and [q] was analyzed. A relevant part of the sequences is shown in Fig. 8. The triangles indicate positions at which these sequences are different. The differences correspond entirely to the ones predicted from the sequences of the M genes of viruses A, E and Q, respectively (Bellini et al., 1985; Cattaneo et al, 1988b). The analysis of a large part of the region in which the three M genes differ (about 800 nucleotides, data not shown) revealed not a single deviation from the sequences expected. The same was true for the hybrid viruses carrying only the first half of the SSPE-derived M genes (results not shown).

Taken together, examples 4 and 5 show that the modified bluescript vector plasmids containing full length MV cDNA inserts, in conjunction with analogous plasmids containing shorter MV cDNA segments, are very suitable for genetic manipulations. All plasmids grow to high copy numbers in the E. Coli strain used; so far no evidence of a single mutation during the multiple ligation and cloning steps has been observed.

Example 6: Insertion of the framework for an additional transcription unit after genes P and H into genomic MV cDNA.

One of the most convenient ways to express proteins of other pathogens from MV vaccine vectors would be to create in the measles genome an additional, seventh transcription unit suitable for insertion of any desired protein coding region. Such an insertion would however undoubtedly result in a somewhat lower transcription rate of all the transcription units downstream of the insert. We chose to attempt the creation of additional transcription units by inserting a second copy of the intercistronic region at the border of genes N and P into the nontranslated 3' terminal region of either genes P and H. The insert, a synthetic DNA segment of 82 nucleotides length (Figure 9, bottom, designated as chip), contains 66 nucleotides completely identical to the N/P gene boundary (28 nucleotides of the 3' nontranslated region of the N gene from position 1718, the nontranscribed 3 nucleotides CTT and 35 nucleotides of the 5' nontranslated region of the P gene until position 1783), followed by a short stretch of nucleotides containing the recognition sites of the restriction endonucleases MluI and NruI, unique for the entire vector bearing the MV genomic cDNA. Provided that viruses can be produced by our procedure from the modified cDNAs, they are expected to express an additional short RNA containing 35 nucleotides identical to the 5' terminal region of the P mRNA, 13 nucleotides mirroring the unique restriction sites, followed, in the two constructs attempted, by either 29 nucleotides according to the 3' terminal region of the P gene or 34 nucleotides according to the analogous region of the H gene; note that these short transcripts are expected to be polyadenylated.

The constructions (see Figure 9) were carried out using a similar strategy as described in Example 4 :

EP 0 440 219 A1

in a first step the short plasmids pePMF2 and peFHL, respectively, cleaved at their unique SpeI restriction sites, were used to introduce the chip constituting the framework for the additional transcription unit. The chip was assembled (by annealing) from four synthetic oligonucleotides (positive strand elements, 36 and 46 nucleotides and negative strand elements, 40 and 42 nucleotides; junction sites indicated by arrowheads in Figure 9, bottom). The protruding single-stranded regions on both sides of the chip fit into the 5' overhangs of the SpeI recognition site; ligation of the chip into the plasmids in the desired orientation leads to the recreation of a SpeI cleavage recognition site at the righthand border of the chip, in contrast to the lefthand border, where only a sequence containing 5 of the 6 nucleotides required for SpeI recognition is formed. This allowed easy selection of the plasmids containing the chip in the desired orientation; the expected sequence in and around the chip was confirmed by sequence determination. In a second step, two plasmids containing the complete MV cDNA with chips inserted in the 3' terminal region of either gene P (peMV(-)chipP) or gene H (peMV(-)chipH) were generated by three-fragment-ligations. A long acceptor fragment obtained by cleavage with SacII and SpeI from plasmid peMV(-) (involving a short 3' terminal portion of gene H, the entire L gene, the modified bluescript vector sequence linking the two extremities of the genomic MV cDNA insert, the entire N and a 5' terminal portion of the P gene) was used for both constructs. Two fragments spanning the region between SacII and NarI sites, recovered from pePMF2 or its derivative pePMF2chip, and NarI - SpeI fragments from peFHL or its derivative peFHLchip, in the appropriate combinations, were ligated to the acceptor fragment.

The final constructs were shown with our helper cell system to give rise to replicating MV yielding normal titers of infectious virions. As for the other reconstructed viruses described above, it was important to ascertain whether the rescued virus genomes agreed with the expected sequences. For this purpose, we tested by PCR amplification whether an RNA segment according to the chip inserted into the plasmids was still present in the RNA genomes and their complementary RNA involved in genome replication. Therefore, RNA was isolated from cells infected with the recovered viruses and reverse transcriptase reactions were primed with oligonucleotides of positive and negative polarity, mapping 50 - 100 nucleotides upstream and downstream, respectively of the regions of interest. The reverse transcriptions were followed by 25 cycles of PCR amplification, using the same oligonucleotides as for reverse transcription. The PCR reaction products around the P/M boundary were expected to be 167 nucleotides long in case of the unmodified region and 249 nucleotides in case of the inserted chip; for the H/L boundary the respective values were 131 and 213 nucleotides. As comparison for the PCR product length, DNA of the two constructed plasmids giving rise to the viruses was used as template. Figure 10 shows the analysis of the PCR products by agarose gel electrophoresis, confirming that the PCR products derived from MV genomic RNA templates are of identical size as the products derived from the plasmid templates.

Thus, the reconstructed viruses can support the insertion of a segment of 82 nucleotides in the indicated two positions of the genome, apparently without compromizing viral viability.

The present invention demonstrates that cloned DNA corresponding to the genome of a negative strand RNA virus can give rise to a replicating virus. Infectious virus can be recovered from cells microinjected with plasmids which generate either the MV genome or the antigenome. However, only few plaques were recovered from the supernatant of helper cells. Trivial reasons for the initial low production of infectious virus may be the damage of many of the 100-200 plasmid/polymerase complexes injected per cell either by the microinjection procedure or the action of cellular nucleases or proteases. In addition, MV genomes containing 2-5 additional nucleotides at each end likely require trimming *in vivo* to the precise genomic length before starting productive replication, as seems to the case for all the infectious RNAs examined until now (Gilvarg et al., 1975; Taniguchi et al., 1978; Ahlquist et al., 1984; Dawson et al., 1986; Meshi et al., 1986; Dasmahapatra et al., 1986; Rice et al., 1987). Most importantly, the presence of nonfunctional envelope proteins in helper cells could prevent efficient formation of infectious viral particles, since the envelope of virus particles budding from microinjected IP-3-Ca cells probably contains a mixture of functional M, F and H proteins, produced *de novo* from copies of the microinjected MV genome, and nonfunctional proteins, present in relatively high abundance both within as on the surface of helper cells. Furthermore, many of the budding virus particles probably enclose defective IP-3-Ca genomes. Only virions containing both a sufficient amount of functional envelope proteins and an Edmonston-Re genome, will be able to interact with the receptors on Vero cells, to fuse their envelope with the cellular membrane, and then to start lytic infection.

As expected, after two passages in Vero cells, reconstituted viruses generated titers by orders of magnitude higher than after microinjection, but not significantly lower than those reached by the Edmonston strain. Analysis of the 5' ends of the genome and antigenome of these reconstituted virus by primer extension revealed that these ends were identical to those of the Edmonston strain virus, confirming that the additional nucleotides flanking the genomic sequence in the primary transcript from the cloned cDNA are

14

removed precisely in vivo by a mechanism not known so far, as it has shown to be the case for all positive-strand RNA viruses expressed from cloned cDNA.

The establishment of a replication system based on cloned cDNA is particularly important for MV because it will now be possible to use recombinant DNA technology in the study of a virus for which only rudimentary genetics and *in vitro* transcription/replication systems are available (Rager-Zisman et al., 1984; Ray and Fujinami, 1987). The development of similar systems will be of great advantage in the study of other nonsegmented, negative strand RNA viruses of medical relevance, such as mumps virus, respiratory syncytial virus, human parainfluenza viruses or rabies virus. For some of these viruses, cell lines infected with conditionally lethal mutants could provide helper function. For other viruses, helper cell lines in which functional genes or synthetic mRNAs have been introduced could be used.

Infectious full length or defective RNAs produced from cDNA clones as described here will also open new experimental possibilities for the study of model negative-strand RNA viruses such as vesicular stomatitis virus or Sendai virus, when used in combination with existing complementation systems (Chattopadhyay and Banerjee, 1987; Meier et al., 1987; Whitt et al., 1989; Gotoh et al., 1989). Even segmented negative-strand RNA viruses like influenza can be rescued by the method of the present invention, using all eight genome segment sequences in the form of cloned cDNAs as described herein for MV.

## MATERIALS AND METHODS

### Cells and viruses

The Edmonston vaccine MV strain was propagated in HeLa (S3) suspension cell cultures and titered on Vero cell monolayers, basically as described (Udem, 1984). The SSPE-derived IP-3-Ca cell line (Burnstein et al., 1974), was propagated in monolayers essentially as detailed previously (Sheppard et al., 1986).

### Transcription and microinjection

BssHII-linearized plasmids peMV(-) or peMV(+) were incubated for 5 minutes at $40^\circ$ C with T3 polymerase (Genofit, Geneva, Switzerland) or at $37^\circ$ C with T7 RNA polymerase (Pharmacia, Uppsala, Sweden) in a buffer containing 40 mM Tris-HCl pH 7.5, 8 mM $MgCl_2$, 0.1mM GTP and 0.1 mM CTP, but without ATP and UTP, to stop RNA synthesis after 4 and 2 nucleotides, respectively. 1,4-dithio-DL-threitol, spermidine and RNAsin were excluded to avoid potential cell toxicity. The plasmid/phage polymerase complex was then diluted 1 to 5 in 0.15 M KCl and microinjected into the cytoplasm of 100-150 cells in a lawn of IP-3-Ca cells grown on coverslips, using Microinjector 5242 (Eppendorf, Hamburg, FRG). The needles were prepared with a Mecanex needle puller (Grade S.A., Nyon, Switzerland), using glass capillaries of 1.2 mm diameter (Clark Electromedical Instruments, Pangbourne, UK). Injection times and pressures were adjusted to deliver ~0.5 pl solution, containing 100-200 molecules of plasmid/phage polymerase complex. After injection, IP-3-Ca cells were cultivated for up to 14 days, supernatants were collected at different time points and stored at -70$^\circ$ C until they were analysed for infectivity in a plaque assay on Vero cell monolayers.

### RNA extraction, reverse transcription, polymerase chain reaction (PCR) and sequencing

RNA was extracted from infected Hela S3, Vero or IP-3-Ca cells using a modified lithium chloride-urea protocol (Auffray and Rougeon, 1980; Cattaneo et al., 1984). Total RNA (2.5 $\mu$g) was used for reverse transcription, by priming with synthetic oligonucleotides specific for the 3' end of the N and P mRNAs as described (Schmid et al., 1987). One fifth of the cDNA recovered was amplified directly by PCR with Taq polymerase (Saiki et al., 1988) using the following primers: for the N gene
5'-(862)GAAATGATATGTGACATTGATAC(884, + strand), and
5'-(1741)TTATAACAATGATGGAGGGTAGGCG (1717, - strand). For the P gene:
5'-(1747)(CG)TTAGGAACCAGGTCCACAGAG (1767, + strand), and
5'-(3399)TTATAATGGATTTAGGTTGTACTAGTTG (3372, - strand). Nucleotides in parenthesis are not colinear with the MV genome; positions are indicated as in Cattaneo et al. (1989b), EMBL/Genbank accession number X 16565-9. Cycles of 1'94$^\circ$ C, 2'50$^\circ$ C and 10' 70$^\circ$ C were repeated 30 times. The amplified fragments were purified on a low melting agarose gel and extracted with Gene Clean (Bio 101, La Jolla, California). Sequencing with two of the primers described above, Sequenase and $^{35}$S-labelled nucleotides was done according to a modification of the dideoxy chain termination protocol allowing direct sequencing of PCR products (Winship, 1989), except that MgCl$_2$ and NaCl were added to the labelling mix only after DNA denaturation.

Additional methods related to recombinant DNA technology

Maniatis et al. (1982, 1989)

REFERENCES

Ahlquist, P., French, R., Janda, M. and Loesch-Fries, L.S. (1984) Proc. Natl. Acad. Sci. USA 81, 7066-7070

Auffray, C. and Rougeon, F. (1980) Eur. J. Biochem. 107, 303-314

Bellini, W.J., Englund, G., Rozenblatt, S., Arnheiter, H. and Richardson, C.D. (1985) J. Virol. 53, 908-919

Billeter, M.A. and Cattaneo, R. (1990) In Kingsbury, D. (ed), The Paramyxoviruses. Plenum Press, New York, in press

Burnstein, T., Jacobsen, L.B., Zeman, W. and Chen, T.T. (1974) Infect. Immun. 10, 1378-1382

Cattaneo, R., Will, H. and Schaller, H. (1984) EMBO J. 3, 2191-2196

Cattaneo, R., Rebmann, G., Schmid, A., Baczko, K., ter Meulen, V. and Billeter, M.A. (1987) EMBO J. 6, 681-688

Cattaneo, R., Schmid, A., Billeter, M.A., Sheppard, R.D. and Udem, S.A. (1988a) J. Virol. 62, 1388-1397

Cattaneo, R., Schmid, A., Eschle, D., Baczko, K., ter Meulen, V. and Billeter, M.A. (1988b) Cell 55, 255-265

Cattaneo, R., Kaelin, K., Baczko, K. and Billeter, M.A. (1989a) Cell 56, 759-764

Cattaneo, R., Schmid, A., Spielhofer, P., Kaelin, K., Baczko, K., ter Meulen, V., Pardowitz, J., Flanagan, S., Rima, B.K., Udem, S.A. and Billeter, M.A. (1989b) Virology 173, 415-425

Chattopadhyay, D. and Banerjee, A.K. (1987) Cell 49, 407-414

Cress, D.E, Kiefer, M.C. and Owens, R.A. (1983) Nucleic Acids Res. 11, 6821-6835

Dasmahapatra, B., Dasgupta, R., Saunders, K., Selling, B., Gallagher, T. and Kaesberg, P. (1986) Proc. Natl. Acad. Sci. USA 83, 63-66

Dawson, W.O., Beck, D.L., Knorr, D.A. and Grantham, G.L. (1986) Proc. Natl. Acad. Sci. USA 83, 1832-1836

Evans, D.J., Mc Keating, J., Meredith, J.M., Burke, K.L.,Katrak, K., John, A., Ferguson, M., Minor, P.D., Weiss, R.A., amd Almond, J.W. (1989) Nature 339, 385-389

Gilvarg, C., Jockusch, H. and Weissmann, C. (1975) Biochem. Biophys. Acta 414, 341-348

Gotoh, H., Shioda, T., Sakai, Y., Mizumoto, K. and Shibuta, H. (1989) Virology 171, 434-443

Luytjes, W., Krystal, M., Enami, M., Parvin, J.D., and Palese, P. (1989) Cell 59, 1107-1113.

Maniatis, T., Fritsch, E.F. and Sambrook, J. (1982) Molecular cloning. A laboratory manual. Cold Spring Harbor Laboratory, New York; also: 2nd edition: Sambrook, J., Fritsch, E.F., and Maniatis (1989)

Meier, E., Harmison, G.G. and Schubert, M. (1987) J. Virol. 61, 3133-3142

Meshi, T., Ishikawa, M, Motcyoshi, F., Semba, K. and Okada, Y. (1986) Proc. Natl. Acad. Sci. USA 83, 5043-5047

Niacin, E., Panicali, D., and Paoletti, E. (1982) Proc. Natl Acad. Sci. USA 79, 1593-1596

Racaniello V.R. and Baltimore D. (1981) Science 214, 916-919

Rager-Zisman, B., Egan, J.E., Kress, Y. and Bloom, B.R. (1984) J. Virol. 51, 845-855

Ray, J. and Fujinami, R.S. (1987) J. Virol. 61, 3381-3387

Rice, C.M., Levis, R., Strauss, J.H. and Huang, H.V. (1987) J. Virol. 61, 3809-3819

Saiki, R.K., Gelfand, D.H., Stoffel, S., Scharf, S.J., Higuchi, R., Horn, G.T., Mullis, K.B. and Erlich, H.A. (1988) Science 239, 487-491

Schmid, A., Cattaneo, R. and Billeter, M.A. (1987) Nucleic Acids Res. 15, 3987-3996

Sheppard, R.D., Raine, C,S., Bornstein, M.B., and Udem, S.A. (1986) Proc. Natl. Acad. Sci. USA 83, 7913-7917

Simon, A.E., Engel, H., Johnson, R.P. and Howell, S.H. (1988) EMBO J. 7, 2645-2651

Spaete, R.R., and Frenkel, N., (1982) Cell 30, 295-304

Spaete, R.R., and Mocarski, E.S. (1987) Proc. Natl. Acad. Sci. USA 84, 7213-7217

Tabler, M. and Sänger, H.L. (1985) EMBO J. 4, 2191-2199

Taniguchi, T., Palmieri, M. and Weissmann, C. (1978) Nature 274, 223-228

ter Meulen, V., Stephenson, J.R. and Kreth, H.W. (1983) Compr. Virol. 18, 105-159

Udem, S.A. (1984) J. Virol. Methods 8, 123-136

Weir P.J., Bajszar, G., and Moss, B. (1982) Proc. Natl. Acad. Sci. USA 79, 1210-1214

Whitt, M.A., Chong, L. and Rose, J.K. (1989) J. Virol. 63, 3569-3578

Winship, P.R. (1989) Nucleic Acids Res. 17, 1266

Xiong, C., Levis, R., Shen, P.,Schlesinger, S., Rice, C.M., and Huang, H.V. (1989) Science 243, 1188-1191

**Claims**

EP 0 440 219 A1

1. A cDNA molecule for the production of negative-strand RNA virus comprising
   (a) the entire genomic or anti-genomic sequence of a non-segmented negative-strand RNA virus, operatively linked to
   (b) an expression control sequence.

2. The cDNA molecule according to claim 1, wherein the expression control sequence (b) is an RNA polymerase promoter.

3. A vector containing a cDNA molecule according to claim 1 or 2.

4. The vector according to claim 3, containing an expressible DNA fragment which replaces a DNA region of said cDNA molecule or provides additional genetic information.

5. The vector according to claim 4, characterized in that the expressible DNA fragment is inserted into a region of said cDNA encoding a viral protein, said insertion being effected in a manner maintaining the reading frame and permitting the expression of said DNA fragment under the control of the signal sequences of said viral protein.

6. The vector according to claim 4, characterized in that the expressible DNA fragment is inserted into a non-coding region of said cDNA and flanked by viral signal sequences or heterologous signal sequences controlling the expression of said DNA fragment.

7. The vector according to any one of claims 3 to 6, which is capable of replicating in a prokaryotic host.

8. The vector according to any one of claims 3 to 6 which is capable of replicating in a eukaryotic host.

9. The vector according to any one of claims 3 to 8, wherein said expressible DNA fragment is a DNA fragment being homologous or heterologous with respect to the negative-strand RNA virus and encoding at least one immunogenic epitope.

10. The vector according to claim 9, wherein said expressible DNA fragment encodes at least one immunogenic epitope of a pathogen.

11. The vector according to claim 10, wherein said expressible DNA fragment is derived from a virus, a bacterium, or a parasite.

12. The vector according to any one of claims 3 to 11, wherein said expressible DNA fragment encodes an immunogenic epitope being able to elicit a protective immune response.

13. A prokaryotic or eukaryotic host cell transformed with a vector according to any one of claims 3 to 12.

14. A helper cell containing a vector according to any one of claims 3 to 12, said helper cell providing the viral proteins necessary for encapsidation, transcription and replication of RNA resulting from transcription of the DNA contained in the vector, and allowing the assembly of infectious negative-strand RNA viruses.

15. An infectious negative-strand RNA virus strain isolated from the helper cell of claim 14.

16. A method for the preparation of recombinant vaccine strains of negative-strand RNA virvses, comprising the steps of:
    (a) introducing a recombinant vector according to any one of claims 3 to 12 as a complex with an RNA polymerase into competent helper cells producing the viral proteins required for the assembly of infectious virus of the negative-strand RNA virus; and
    (b) recovering the assembled infectious negative-strand RNA viruses.

17. The method according to claim 16, wherein said recombinant vector is obtainable by a method

17

comprising the following steps:

(A) preparing a full-length genomic cDNA molecule by assembly of cDNA clones derived from mono- and/or polycistronic viral mRNA;

(B) inserting said cDNA of step (a) into a vector under the control of an expression control sequence, preferably an RNA polymerase promoter, so as to allow the initiation of transcription of said cDNA by said control sequence;

(C) incorporating into a viral protein-encoding region of said cDNA a DNA fragment in reading frame so as to be expressible under the control of the signal sequences of said viral protein or incorporating said DNA fragment into a non-coding region of said cDNA so as to be expressible under the control of a flanking viral or heterologous signal sequence, wherein said expressible DNA fragment replaces a DNA region of said cDNA molecule or provides additional genetic information;

(D) cloning the obtained recombinant vector in a microorganism; and

(E) isolating said recombinant vector.

18. Vaccine containing an infectious negative-strand RNA virus strain according to claim 15 or obtainable by the method according to claim 16 or 17, optionally in combination with a pharmaceutically acceptable carrier and/or diluent.

# Scheme for cDNA cloning of negative-strand RNA viral genomes and expression of the cDNA as replicating virus

RNA from cells infected with a negative-strand RNA virus

↓

Specific cDNA cloning by use of chemically synthesized oligonucleotides

↓

Assembly of overlapping clones to full length viral genomic or antigenomic
sequences in a plasmid vector closely linked to expression control
sequences (eg. T7 or T3 promoters)

↓

Linearization of the DNA by cleavage immediately downstream the viral sequence

↓

Formation of committed complexes by incubation with RNA polymerase and two
or three nucleoside triphosphates

↓

Introduction into helper cells (eg. IP-3-Ca in case of MV) providing the viral
proteins necessary for encapsidation of the nacent RNA as well as
transcription and replication of the completed viral genomes

↓

Isolation of excreted virus and multiplication in cells (eg. human diploid lines)

**Figure 1**

Figure 2

20

Figure 3

Figure 4

Figure 5

Figure 6

Figure 7

Figure 8

Figure 9

Figure 10

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X,D | NUCLEIC ACIDS RESEARCH, vol. 15, no. 10, 1987, pages 3987-3996, IRL Press Ltd, Oxford, GB; A. SCHMID et al.: "A procedure for selective full length cDNA cloning of specific RNA species <br> * Whole document, especially figure 1; page 3995, last paragraph before Acknowledgments * <br> - - - | 1-13 | C 12 N 15/45 <br> C 12 N 15/47 <br> C 12 N 5/10 <br> A 61 K 39/165 <br> A 61 <br> K 39/205 <br> C 12 N 7/00 |
| A | EP-A-0 237 686   (INSTITUT PASTEUR)(23-09-1987) <br> * Whole document * <br> - - - | 1-18 | |
| P,X | THE EMBO JOURNAL, vol. 9, no. 2, February 1990, pages 379-384, Oxford University Press, Eynsham, Oxford, GB; I. BALLART et al.: "Infectious measles virus from cloned cDNA" <br> - - - - - | 1-18 | |

TECHNICAL FIELDS
SEARCHED (Int. Cl.5)

C 07 K
C 12 N
A 61 K

The present search report has been drawn up for all claims

| Place of search | Date of completion of search | Examiner |
|---|---|---|
| The Hague | 08 May 91 | CUPIDO M. |